# FASCICULE DE BREVET EUROPEEN

(11) **EP 0 958 290 B1**
(45) Date de publication et mention de la délivrance du brevet: **31.07.2002**
(21) Numéro de dépôt: 98902058.1
(22) Date de dépôt: 12.01.1998
(51) Int. Cl.: C07D 409/14, C07D 401/12, C07D 401/14, C07D 211/42, C07D 207/12, A61K 31/445

(54) **DERIVES DE LA 1,4-DIHYDROPYRIDINE ET LEUR UTILISATION EN THERAPEUTIQUE**
NEUE 1,4-DIHYDROPYRIDINDERIVATE UND DEREN THERAPEUTISCHE ANWENDUNG
1,4-DIHYDROPYRIDIN DERIVATIVES AND THEIR USE IN THERAPY

(30) Priorité: 21.01.1997 FR 9700577
(43) Date de publication de la demande: 24.11.1999
(73) Titulaire: LABORATOIRE L. LAFON, 94701 Maisons Alfort (FR)
(72) Inventeur: DURET, Gérard, F-92100 Boulogne Billancourt (FR); GLAUERT, Gérard, F-77120 Saints (FR); HENRY, Marguerite, F-92100 Boulogne Billancourt (FR)
(74) Mandataire: Bernasconi, Jean Raymond
(86) Numéro de dépôt international: FR9800042
(87) Numéro de publication internationale: WO9831680

(56) Documents cités:
- EP-A- 0 494 816

## Description

La présente invention concerne de nouveaux dérivés de la 1,4-dihydropyridine.

Différents dérivés de 1,4-dihydropyridine ont déjà été décrits. Ainsi, notamment, dans FR 2 218 107, on a déjà décrit différents esters de l'acide 2,6-diméthyl-4-(3-nitrophényl)-5-méthoxycarbonyl-1,4-dihydropyridine-3-carboxylique et notamment l'ester 2[méthyl(phénylméthyl) amine]éthylique connu sous le nom de nicardipine.

On a par ailleurs décrit dans EP-A-0494 816 d'autres dérivés de 1,4-dihydropyridine présentant en position 3 une chaîne du type: 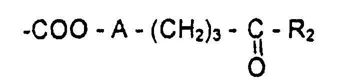 dans laquelle :
A représente un groupe choisi parmi les groupes de formule: 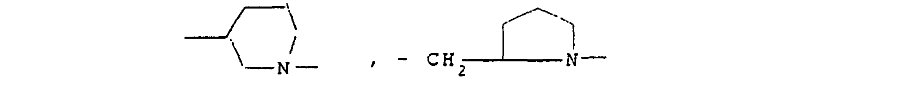
et R₂ représente un groupe choisi parmi les groupes 2,4,6-triméthoxyphényle, 2-thiényle et phényle.

La présente invention vise à fournir de nouveaux dérivés de 1,4-dihydropyridine qui présentent une marge thérapeutique améliorée par rapport à celle de composés répondant à la formule décrite dans EP-A-0 494 816.

La présente invention a ainsi pour objet des composés de formule : 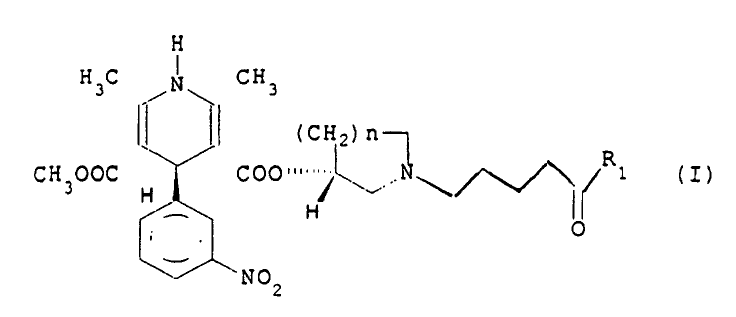 dans laquelle :
n = 1 ou 2, et
R₁ est un groupe choisi parmi les groupes 2,4,6-triméthoxyphényle, 2-thiényle et 2-pyrrolyle,
et leurs sels d'addition avec des acides pharmaceutiquement acceptables.

La présente invention a plus particulièrement pour objet
le (4S, 3'R)(-) 2,6-diméthyl-4-(3-nitrophényl)-5-méthoxycarbonyl-1,4-dihydro- pyridine-3-carboxylate de N-[4-(2-thiénoyl)butyl]pipéridine-3'-yle,
le (4S, 3'R)(-) 2,6-diméthyl-4-(3-nitrophényl)-5-méthoxycarbonyl-1,4-dihydropyridine-3-carboxylate de N-[4-(2,4,6-triméthoxybenzoyl)-butyl]pyrrolidine-3'-yle,
le (4S, 3'R)(-) 2,6-diméthyl-4-(3-nitrophényl)-5-méthoxycarbonyl-1,4-dihydropyridine-3-carboxylate de N-[5-(1H-pyrrole-2-yl)-5-oxopentyl]-pipéridine-3'-yle,
et leurs sels d'addition avec des acides pharmaceutiquement acceptables.

Les « sels d'addition avec des acides pharmaceutiquement acceptables » désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques.

Les composés de formule I peuvent être obtenus par la réaction de l'acide de formule : 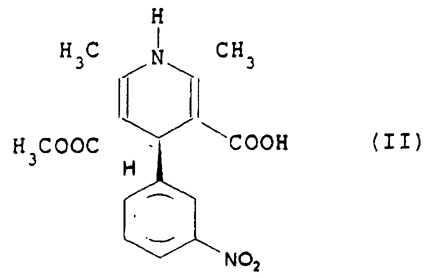 avec un alcool de formule 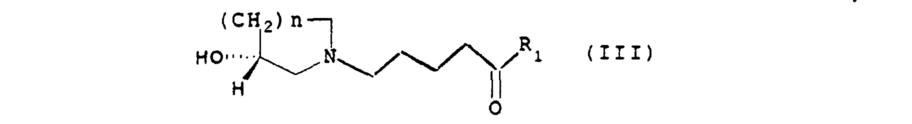 dans laquelle n et R₁ ont la signification donnée précédemment.

Les sels d'addition s'obtiennent de façon classique par réaction du composé de formule I avec un acide pharmaceutiquement acceptable dans un solvant approprié. Inversement, les bases peuvent être obtenues à partir des sels d'addition par traitement par une base forte.

L'acide de formule II peut être préparé comme décrit dans EP-A-0 494 816 ou comme décrit dans EP-A-0 680 952.

Les alcools de formule III peuvent être obtenus par réaction d'un composé de formule : 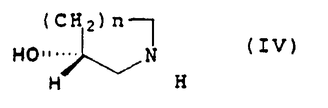 avec un dérivé chloré de formule

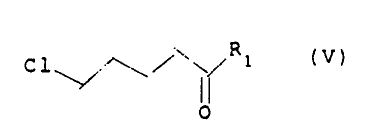

Les exemples suivants illustrent la préparation des composés selon l'invention.

### Exemple 1

### Préparation de (4S, 3'R)- (-) 2,6-diméthyl-4-(3-nitrophényl)-5-méthoxycarbonyl-1,4-dihydropyridine-3-carboxylate de N-[4-(2-thiénoyl)butyl]pipéridine-3'-yle (CRL 42 249)

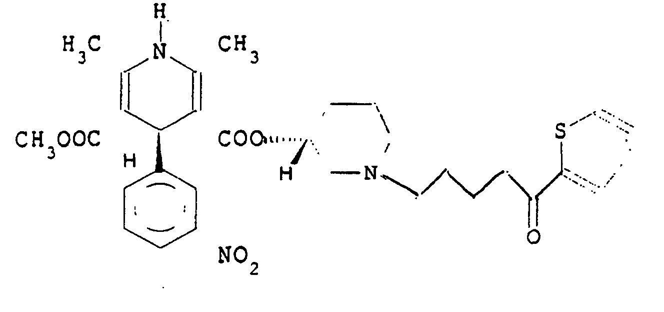

### a) Préparation du 2(5-chloropentanoyl)-thiophène

On introduit par fractions en 15 mn : 16 g (0,120 mole) de chlorure d'aluminium, au sein d'une solution maintenue vers +5° de 20,2 g (0,240 mole) de thiophène et de 15;5 g (0,100 mole) de chlorure de 5-chloro valéryle et on agite 5 h à la température ambiante. Après élimination du surnageant par décantation, le milieu réactionnel est repris par 120 ml d'une solution d'acide chlorhydrique 3N et extrait par du chloroforme.

La phase organique est lavée par de l'eau, séchée sur sulfate de sodium sec et le solvant évaporé pour donner 16,5 g d'une huile brune orangée.
Rendement : 81,65 %.

### b) Préparation du (R)2-[5-(3-hydroxy-pipéridino pentanoyl]thiophène, chlorhydrate.

Au sein d'une suspension au reflux de 8,1 g (0,08 mole) de (R) 3-hydroxy pipéridine préparé comme décrit par H. Siertsson et al. (J. Med. Chem. 15, 1085, 1972) et de 12,7 g (0,12 mole) de carbonate de sodium dans 20 ml d'acétonitrile, on coule en 45 mn, une solution de 20,3 g (0,10 mole) du composé obtenu en a) dans 10 ml d'acétonitrile et on maintient le reflux 1 h. Le milieu réactionnel est dilué par de l'acétate d'éthyle, lavé par de l'eau et séché sur sulfate de sodium sec.

On traite la phase organique par de l'isopropanol chlorhydrique et on purifie le précipité par une cristallisation dans l'isopropanol pour donner 17 g d'une poudre légèrement rose.
Rendement : 70 %
F₍ᵢₙₛₜ₎Kofler : 143° C
RMN 200 MHz -TF-¹H(CD₃OD) : 7,3-8,1 (m, 3H, thiényl-H); 4,3 (m, 1H, 3-pipéridinyl-H); 1,6-3,7 (m, 16H, CH₂).

### c) Préparation de (4S, 3'R)- (-) 2,6-diméthyl-4-(3-nitrophényl)-5-méthoxycarbonyl-1,4-dihydropyridine-3-carboxylate de N-[4-(2-thiénoyl)butyl]pipéridine-3'-yle

On agite pendant 3 jours à la température ambiante, une suspension de 16,6 g (0,050 mole) de (4-R)-(-)acide 5-méthoxycarbonyl-2,6-diméthyl-4-(3-nitrophényl)-1,4-dihydropyridine-3-carboxylique, de 13,4 g (0,050 mole) de produit obtenu en b), de 3,05 g (0,025 mole) de diméthyl aminopyridine et de 20,6 g (0,100 mole) de dicyclohexylcarbodiimide dans 400 ml de toluène. L'insoluble est éliminé par filtration, le solvant évaporé et le résidu repris par du chlorure de méthylène que l'on lave successivement par une solution de soude 2N, une solution d'acide chlorhydrique 2N et une solution de bicarbonate de potassium à 5 %. On sèche la phase organique sur sulfate de sodium sec et on évapore le solvant sous pression réduite.

Le résidu est purifié par un passage sur 200 g de silice (chromatographie flash) en éluant par le mélange chlorure de méthylène-95/isopropanol-5 pour donner 18,6 g d'une poudre jaune amorphe.
Rendement : 64 %
[α]_{D}-23(c = 1,284, MeOH)
RMN 200 MHz -TF-¹H(CDCl₃) : 7,1-8,15 (m, 7H, aromatique-H); 5,1 (s, 1H, 4-dihydropyridyl-H); 4,75 (m, 1H, 3-pipéridinyl-H); 3,65 (s, 3H, COOCH₃); 1,2-3(m, 16H-CH₂-), 2,3 (s, 1H, NH).

### Exemple 2

### Préparation de (4S, 3'R)-(-) 2,6-diméthyl-4-(3-nitrophényl)-5-méthoxycarbonyl-1,4-dihydropyridine-3-carboxylate de N-[4-(2,4,6-triméthoxybenzoyl)- butyl]pyrrolidine-3'-yle (CRL 42 290)

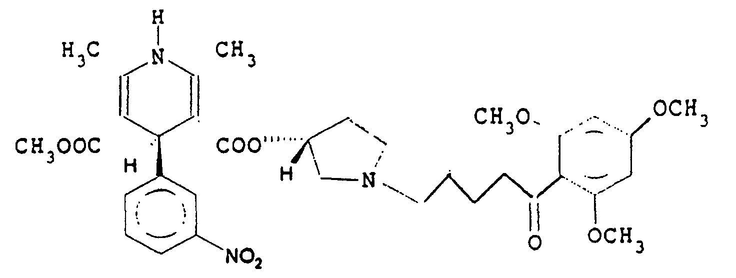

### a) Préparation de 1-(2,4,6-triméthoxybenzoyl)-4-chlorobutane

Au sein d'une solution maintenue vers +5° C de 50,4 g (0,3200 mole) de 1,3,5-triméthoxybenzène et de 50,2 g (0,324 mole) de chlorure de chloro-5 valéryle dans 225 ml de benzène, on coule en 1 h 30 une solution de 91 g(0,384 mole) de tétrachlorure d'étain dans 112,5 ml de benzène. On agite une nuit à la température ambiante et on jette le milieu réactionnel sur 325 ml d'eau glacée et 75 ml d'acide chlorhydrique 12 N. On décante la phase organique, que l'on lave par de l'eau et que l'on sèche sur sulfate de sodium sec.

Le résidu huileux est purifié par un lavage dans l'hexane, pour donner 84 g d'une poudre blanche.
F < 50° C
Rendement = 97,7 %
RMN 200 MHz -TF-¹H(CDCl₃) : 6,1 (s, 2H, aromatique-H), 3,9 (s, 3H, méthoxy-H); 3,7 (s, 6H, méthoxy-H); 3,5 (t, 2H, CH₂Cl); 2,7 (t, 2H, CH₂CO); 1,8 (m, 4H, CH₂);

### b) Préparation de (R)-(-) 1-(2,4,6-triméthoxybenzoyl)-4-(3-hydroxypyrrolidino)butane, chlorhydrate

Au sein d'une solution au reflux de 17 g (0,1955 mole) de (R) 3-hydroxypyrrolidone dans 20 ml de toluène, on coule en 1 h 15, une solution de 28 g (0,0977 mole) du produit obtenu en a) dans 55 ml de toluène, on maintient au reflux 30 mn et on dilue le milieu réactionnel par de l'acétate d'éthyle. On lave par de l'eau, on extrait par une solution d'acide chlorhydrique dilué, on alcalinise la phase aqueuse par de la soude concentrée et on extrait à nouveau par de l'acétate d'éthyle.

Après séchage de la phase organique sur sulfate de sodium sec, on traite par de l'isopropanol chlorhydrique.

Le précipité obtenu est purifié par une cristallisation dans l'isopropanol pour donner 15,2 g d'une poudre légèrement mauve, soluble dans l'eau.
F. inst. (Kofler) = 125° C
Rendement = 41,65 %
RMN 200 MHz -TF-¹H(CD₃OD) : 6,2 (s, 2H, aromatique-H); 3,85 (s, 3H, méthoxy-H); 3,75 (s, 6H, méthoxy-H); 3-3,5 (m, 6H, N-CH₂); 2,8 (t, 2H, CH₂-CO); 1,6-2,1 (m, 6H, CH₂).

### c) Préparation de (4S, 3'R)-(-) 2,6-diméthyl-4-(3-nitrophényl)-5-méthoxycarbonyl-1,4-dihydropyridine-3-carboxylate de N-[4-(2,4,6-triméthoxybenzoyl)- butyl]pyrrolidine-3'-yle

Au sein d'une suspension de 8 g (0,024 mole) de (4-R)-(-)acide 5-méthoxycarbonyl-2,6-diméthyl-4-(3-nitrophényl)-1,4-dihydropyridine-3-carboxylique et de 8,1 g (0,024 mole) du produit obtenu en b) sous forme de base, en présence de 1,5 g (0,012 mole) de diméthyl aminopyridine dans 140 ml de toluène, on coule une solution de 9,9 g (0,048 mole) de dicyclohexylcarbodiimide dans 60 ml de toluène. On agite 4 jours à la température ambiante, on élimine le précipité par filtration et on évapore le solvant sous pression réduite. Le résidu est repris par du chloroforme que l'on lave par de la soude 2 N, de l'acide chlorhydrique 2 N et une solution de bicarbonate de potassium à 5 %.

Après séchage sur sulfate de sodium sec et évaporation du solvant, le résidu est purifié par chromatographie flash sur colonne de silice par le mélange chlorure de méthylène-97,5/isopropanol-2,5, pour donner 8,8 g d'une poudre « meringuée » jaune, insoluble dans l'eau.
Rendement = 56,3 %
Rendement total = 22,9 %
[α]_{D}-1,2 (c = 1,252, MeOH)
RMN 200 MHz -TF-¹H(CDCl₃) : 7,2-8,1 (m, 4H, aromatique-H); 6,1 (s, 2H, aromatique-H); 5,1 (m, 2H, 4-dihydropyridyl-H; 3-pyrrolidinyl-H); 3,8 (s, 3H, méthoxy-H); 3,7 (s, 6H, méthoxy-H); 3,6 (s, 3H, COOCH₃); 2,3 (s, 6H, CH₃); 1,4-2,9 (m, 14 H, CH₂).

### Exemple 3

### Préparation de (4S, 3'R)- (-) 2,6-diméthyl-4-(3-nitrophényl)-5-méthoxycarbonyl-1,4-dihydropyridine-3-carboxylate de N-[5-(1H-pyrrole-2-yl)-5-oxopentyl]-pipéridine-3'-yle (CRL 42 547)

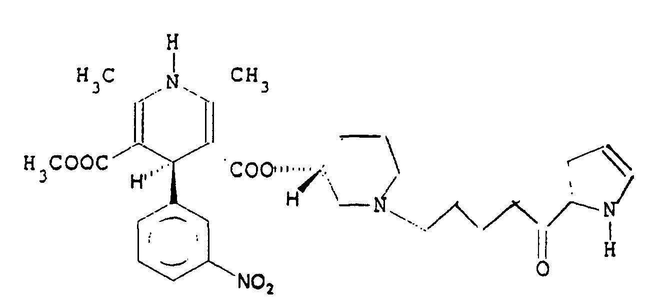

### a) Préparation de 2-(5-chloro pentanoyl)-1H-pyrrole

Au sein d'une solution de 13,4 g (0,200 mole) de pyrrole dans 250 ml de dichloro-1-2 éthane, on coule lentement une solution de 26,7 g (0,200 mole) de chlorure d'aluminium et de 31 g (0,200 mole) de chlorure de chloro-5 valéryle dans 200 ml de 1-2, dichloroéthane. On agite 2 h à la température ambiante et on jette le milieu réactionnel sur de l'acide chlrhydrique glacé. Après extraction par du chlorure de méthylène, on obtient une huile brun rouge.

Ce produit est purifié par un passage sur chromatographie flash de gel de silice 60 et élué par le mélange hexane-90/acétate d'éthyle-10, pour donner 15 g d'une huile légèrement orangée.
Rendement = 40,4 %
RMN 200 MHz -TF-¹H(CDCl₃) : 6,2-7,1 (m, 3H, pyrrole-H); 3,5 (t, 2H, CH₂Cl); 2,8 (t, 2H, CH₂-CO); 1,8 (m, 4H, CH₂).

### b) Préparation de 2-[5-(3-hydroxy pipéridino) pentanoyl]-1H-pyrrole, chlorhydrate

Au sein d'une suspension au reflux de 8,9 g (0,089 mole) de (R)-3-hydroxy pipéridine, de 18,4 g (0,1335 mole) de carbonate de potassium et de 2,2 g (0,01335 mole) d'iodure de potassium dans 18 ml d'acétonitrile on coule en 1 h une solution de 16,5 g (0,089 mole) du produit obtenu en a) dans 20 ml d'acétonitrile et on poursuit le reflux une nuit. On dilue le milieu réactionnel par de l'acétate d'éthyle, on lave par de l'eau et on extrait par une solution d'acide chlorhydrique 2N. La phase aqueuse est alcalinisée par de la soude concentrée et l'insoluble extrait par de l'acétate d'éthyle. La phase organique est traitée par de l'isopropanol chlorhydrique pour donner 20,2 g d'une poudre gris vert.
F. inst. (Kofler) = 188° C
Rendement = 79,2 %
RMN 200 MHz -TF-¹H(CDCl₃) : 6,2-7,1 (m, 3H, pyrrole-H); 4,3 (m, 1H, CH-OH); 3-3,5 (m, 6H, N-CH₂); 2,8 (t, 2H, CH₂-CO); 1,6-2 (m, 8H, CH₂).

### c) Préparation de (4S, 3'R)- (-) 2,6-diméthyl-4-(3-nitrophényl)-5-méthoxycarbonyl-1,4-dihydropyridine-3-carboxylate de N-[5-(1H-pyrrole-2-yl)-5-oxopentyl]-pipéridine-3'-yle

Au sein d'une suspension maintenue sous atmosphère d'azote, de 12,6 g (0,038 mole) de (4-R)-(-)acide 5-méthoxycarbonyl-2,6-diméthyl-4-(3-nitrophényl)-1,4-dihydropyridine-3-carboxylique, et de 9,5 g (0,038 mole) du produit obtenu en b) sous forme de base en présence de 2,3 g (0,019 mole) de diméthyl aminopyridine dans 250 ml de toluène, on coule une solution de 15,7 g (0,076 mole) de dicyclohexylcarbodiimide dans 125 ml de toluène. On agite 7 jours à la température ambiante, on élimine le précipité par filtration et on évapore le solvant sous pression réduite. Le résidu est repris par du chlorure de méthylène que l'on lave par de la soude 2 N, de l'acide chlorhydrique 2 N et une solution de bicarbonate de potassium à 5 %.

Après séchage sur sulfate de sodium sec et évaporation du solvant, le résidu est purifié par chromatographie flash sur colonne de silice par le mélange chlorure de méthylène-95/isopropanol-5, pour donner 14 g d'une poudre « meringuée » jaune, insoluble dans l'eau.
Rendement = 65,3 %
Rendement total = 20,9 %
[α]_{D}-23,7 (c = 1,332, MeOH)
RMN 200 MHz -TF-¹H(CDCl₃) : 7,3-8,1 (m, 4H, aromatique-H); 6,2-7,1 (m, 3H, pyrrole-H); 5,1 (s, 1H, 4-dihydropyridinyl-H); 4,8 (m, 1H, 2-pipéridinyl-H); 3,6 (s, 3H, COOCH₃); 1,3-2,8 (m, 16 H, CH₂); 2,3 (s, 6H, CH₃).

On donnera ci-après des résultats pharmacologiques mettant en évidence les propriétés avantageuses des composés de l'invention.

### 1) Activité anticalcique par mesure de l'affinité pour les sites DHP des ventricules cardiaques de rat

L'affinité des composés pour les sites DHP des ventricules cardiaques, est mesurée à partir d'une préparation membranaire des récepteurs, préparation obtenue après dissection des ventricules, homogénéisation puis double centrifugation (48000 g; 15 min., + 4° C).

Ces préparations membranaires sont mises en contact avec le ligand radioactif spécifique (+) [³H]-PN 200-110 et le composé à étudier, à différentes concentrations. La suspension est agitée 30 minutes à la température de 30° C. La réaction est alors arrêtée par filtration à l'aide d'un système type Harvester. Le filtre est introduit dans une fiole de comptage contenant du liquide scintillant. La radioactivité présente sur chaque filtre est alors mesurée par comptage dans un compteur β à scintillation liquide.

L'intensité de la liaison aux récepteurs membranaires est définie par la concentration (molaire) du composé à tester qui est nécessaire pour déplacer 50 % de la quantité du ligand spécifique (+) [³H]-PN 200-110 préalablement lié aux sites DHP. Cette concentration est la concentration CI₅₀.

Les résultats exprimés en CI₅₀ sont donnés dans le tableau suivant :

| Composé | CI₅₀ |
|---|---|
| Exemple 1 | 1,5.10⁻⁸ |
| Exemple 2 | 7,8.10⁻⁹ |

### 2) Activité anticalcique par mesure de l'antagonisme de la contraction de l'aorte isolée de rat par KCl

La technique mise en oeuvre utilise un anneau de tissu vasculaire artériel prélevé sur l'aorte thoracique du rat puis maintenu en survie dans du tampon Krebs bicarbonaté aéré, et soumis à une tension initiale de 2 g. L'introduction de chlorure de potassium KCI (sous un volume de 300 µl) à la concentration de 5.10⁻²M.I⁻¹ dans le bain de tampon de Krebs génère une contraction soutenue dont l'amplitude (tension isométrique) est antagonisée par l'addition d'une solution du composé étudié de concentrations croissantes, chaque addition étant pratiquée toutes les 5 minutes. On calcule ensuite la concentration molaire du composé étudié qui diminue de 50 % la contraction maximale observée sous KCI. Cette concentration est la concentration inhibitrice 50 % (ou CI₅₀).

Les résultats exprimés en CI₅₀ sont donnés dans le tableau suivant :

| Composé | CI₅₀ |
|---|---|
| Exemple 1 | 1,3.10⁻⁷ |
| Exemple 3 | 1,6.10⁻⁷ |

### 3) Effet hypotenseur chez le rat spontanément hypertendu éveillé

Les animaux reçoivent par voie gastrique des doses croissantes de composé toutes les 90 minutes.

L'effet hypotenseur est évalué par le pourcentage de diminution maximum de la pression artérielle moyenne après administration de chaque dose.

| Composé | Dose mg/kg | | |
|---|---|---|---|
| | 3 | + 10 | +30 |
| Exemple 1 | 0% | - 22 % | - 45 % |
| Exemple 3 | -12 % | - 21 % | - 54 % |

### 4) Effet sur le débit coronaire du chien anesthésié

Les animaux reçoivent par voie IV des doses croissantes de composé toutes les 30 minutes. L'effet sur le débit coronaire est évalué par son pourcentage de variation maximum après chaque dose.

| Dose µg/kg | Composé | | |
|---|---|---|---|
| | Exemple 1 | Exemple 2 | Exemple 3 |
| 5 | 12% | +16% | + 7% |
| +10 | +28% | +14% | +30% |
| +20 | +56% | +34% | +59% |
| +40 | +72% | +78% | +76% |
| +80 | +81% | +68% | +84% |
| +160 | +67% | +59% | +84% |
| +320 | +64% | +33% | +59% |

### 5) Marge thérapeutique

La marge thérapeutique est définie comme étant le rapport entre la dose minimale arythmogène et la dose qui augmente de 50 % le débit coronaire basal (cette dose est appelée ci-après « dose active sur le débit coronaire »).

On a déterminé pour chaque composé chez le chien anesthésié les doses minimales arythmogènes par voie I.V. et on a déterminé le rapport dose arythmogène/dose active sur le débit coronaire (marge thérapeutique).

On donnera ci-après les résultats obtenus :

| Composé | Dose arythmogène/ Dose active sur le débit coronaire |
|---|---|
| Exemple 1 | 640/20 = 32 |
| Exemple 2 | 640/40 = 16 |
| Exemple 3 | 640/20 = 32 |
| Exemple A (comparaison) | 640/640 = 1 |
| Exemple B (comparaison) | 640/640 = 1 |

Les composés de comparaison A et B sont des composés qui ne diffèrent respectivement des composés des exemples 1 et 2 que par la longueur de la chaîne (3 carbones au lieu de 4 carbones entre l'hétérocycle azoté et le groupe 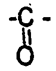 ).

La présente invention a également pour objet des compositions thérapeutiques comprenant à titre de principe actif un composé de formule I ou l'un de ses sels d'addition avec des acides pharmaceutiquement acceptables.

Les compositions thérapeutiques selon l'invention peuvent être administrées à l'homme ou aux animaux par voie orale ou parentérale.

Elles peuvent être sous la forme de préparations solides, semi-solides ou liquides. Comme exemple, on peut citer les comprimés, les gélules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

## Revendications

1. Composés de formule : 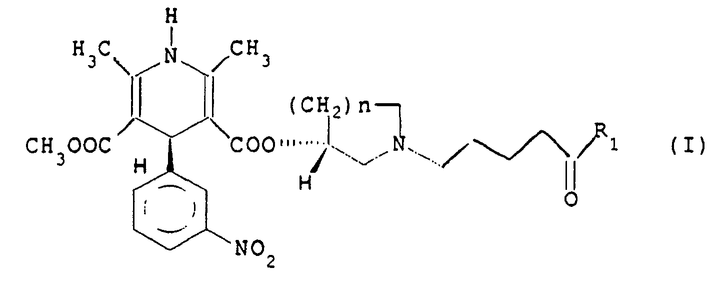 dans laquelle :
n = 1 ou 2, et
R₁ est un groupe choisi parmi les groupes 2,4,6-triméthoxyphényle, 2-thiényle et 2-pyrrolyle,
et leurs sels d'addition avec des acides pharmaceutiquement acceptables.

2. Composé selon la revendication 1, qui est le (4S, 3'R)-(-) 2,6-diméthyl-4-(3-nitrophényl)-5-méthoxycarbonyl-1,4-dihydropyridine-3-carboxylate de N-[4-(2-thiénoyl)butyl]pipéridine-3'-yle, et ses sels d'addition avec des acides pharmaceutiquement acceptables.

3. Composé selon la revendication 1, qui est le (4S, 3'R)-(-) 2,6-diméthyl-4-(3-nitrophényl)-5-méthoxycarbonyl-1,4-dihydropyridine-3-carboxylate de N-[4-(2,4,6-triméthoxybenzoyl)-butyl]pyrrolidine-3'-yle, et ses sels d'addition avec des acides pharmaceutiquement acceptables.

4. Composé selon la revendication 1, qui est le (4S, 3'R)-(-) 2,6-diméthyl-4-(3-nitrophényl)-5-méthoxycarbonyl-1,4-dihydropyridine-3-carboxylate de N-[5-(1H-pyrrole-2-yl)-5-oxopentyl]- pipéridine-3'-yle, et ses sels d'addition avec des acides pharmaceutiquement acceptables.

5. Procédé de préparation d'un composé selon la revendication 1, comprenant la réaction de l'acide de formule 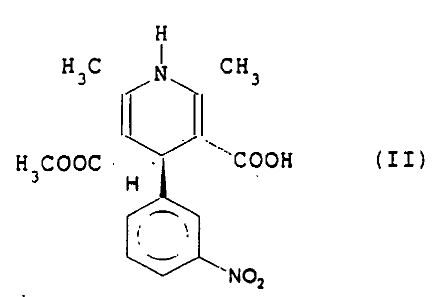 avec un alcool de formule 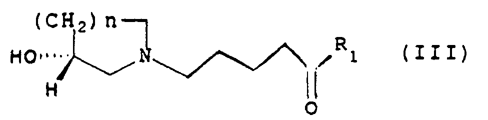 dans laquelle n et R₁ ont la signification donnée précédemment.

6. Composition thérapeutique comprenant, à titre de principe actif, un composé selon l'une quelconque des revendications 1 à 4.

7. Composés de formule 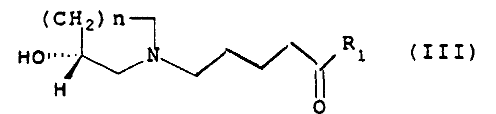 dans laquelle n et R₁ ont la signification donnée à la revendication 1.

## Patentansprüche

1. Verbindungen mit der Formel 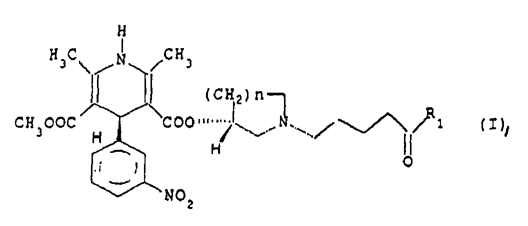 in welcher
n = 1 oder 2 ist und
R₁ eine Gruppe bedeutet, die aus der 2,4,6-Trimethoxyphenyl-, 2-Thienyl- und 2-Pyrrolylgruppe ausgewählt ist, und
ihre Additionssalze mit pharmazeutisch verträglichen Säuren.

2. Verbindung nach Anspruch 1, die N-[4-(2-Thienoyl)-butyl]piperidin-3'-yl-(4S, 3'R)-(-)-2,6-dimethyl-4-(3-nitrophenyl)-5-methoxycarbonyl-1,4-dihydropyridin-3-carboxylat ist, und ihre Additionssalze mit pharmazeutisch verträglichen Säuren.

3. Verbindung nach Anspruch 1, die N-[4-(2,4,6-Trimethoxybenzoyl)-butyl]pyrrolidin-3'-yl-(4S, 3'R)-(-)-2,6-dimethyl-4-(3-nitrophenyl)-5-methoxycarbonyl-1,4-dihydropyridin-3-carboxylat ist, und ihre Additionssalze mit pharmazeutisch verträglichen Säuren.

4. Verbindung nach Anspruch 1, die N-[5-(1H-Pyrrol-2-yl)-5-oxopentyl]-piperidin-3'-yl-(4S, 3'R)-(-)-2,6-dimethyl-4-(3-nitrophenyl)-5-methoxycarbonyl-1,4-dihydropyridin-3-carboxylat ist, und ihre Additionssalze mit pharmazeutisch verträglichen Säuren.

5. Verfahren zur Herstellung einer Verbindung nach Anspruch 1, das die Umsetzung einer Säure mit der Formel 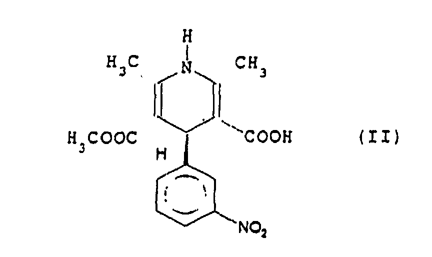 mit einem Alkohol mit der Formel 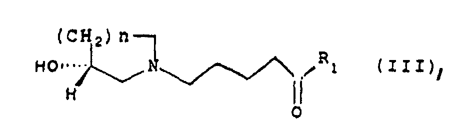 in welcher n und R₁ die oben angegebene Bedeutung haben, umfasst.

6. Therapeutische Zusammensetzung, die als Wirkstoff eine Verbindung nach einem der Ansprüche 1 bis 4 enthält.

7. Verbindungen mit der Formel 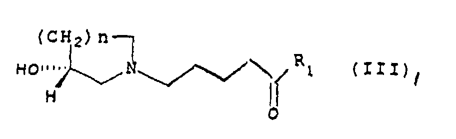 in welcher n und R₁ die im Anspruch 1 angegebene Bedeutung haben.

## Claims

1. Compounds of formula: 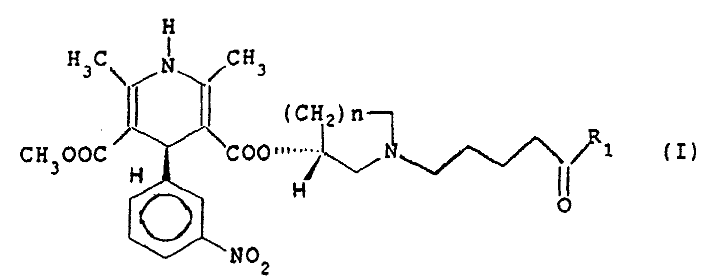 wherein:
n = 1 or 2, and
R₁ is a group selected from among the 2,4,6-trimethoxyphenyl, 2-thienyl and 2-pyrrolyl groups,
and the addition salts thereof with pharmaceutically acceptable acids.

2. Compound according to claim 1 which is N-[4-(2-thienoyl)butyl]piperidin-3'-yl (4S,3'R)-(-) 2,6-dimethyl-4-(3-nitrophenyl)-5-methoxycarbonyl-1,4-dihydropyridine-3-carboxylate and the addition salts thereof with pharmaceutically acceptable acids.

3. Compound according to claim 1 which is N-[4-(2,4,6-trimethoxybenzoyl)-butyl]pyrrolidin-3'-yl (4S,3'R)-(-) 2,6-dimethyl-4-(3-nitrophenyl)-5-methoxycarbonyl-1,4-dihydropyridine-3-carboxylate and the addition salts thereof with pharmaceutically acceptable acids.

4. Compound according to claim 1 which is N-[5-(1H-pyrrol-2-yl)-5-oxopentyl]-piperidin-3'-yl (4S,3'R)-(-) 2,6-dimethyl-4-(3-nitrophenyl)-5-methoxycarbonyl-1,4-dihydropyridine-3-carboxylate and the addition salts thereof with pharmaceutically acceptable acids.

5. Process for preparing a compound according to claim 1, comprising reacting the acid of formula: 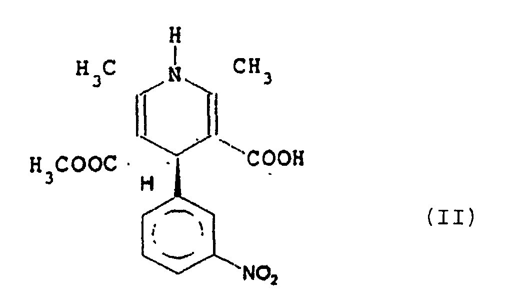 with an alcohol of formula 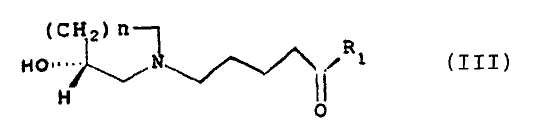 wherein n and R₁ are as hereinbefore defined.

6. Therapeutic composition containing, as active ingredient, a compound according to any one of claims 1 to 4.

7. Compounds of formula 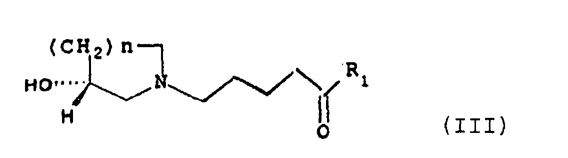 wherein n and R₁ are defined as in claim 1.
